# EUROPÄISCHE PATENTSCHRIFT

(11) **EP 0 512 953 B1**
(45) Veröffentlichungstag und Bekanntmachung des Hinweises auf die Patenterteilung: **07.06.1995**
(21) Anmeldenummer: 92810315.9
(22) Anmeldetag: 29.04.1992
(51) Int. Cl.: C07C 311/16, C07C 303/36, C07C 309/44, C07C 309/86

(54) **Verfahren zur Herstellung von Benzolsulfonamiden**
Process for the preparation of benzene sulfonamides
Procédé pour la préparation de benzène sulfonamides

(30) Priorität: 06.05.1991 CH 1347/91
(43) Veröffentlichungstag der Anmeldung: 11.11.1992
(73) Patentinhaber: CIBA-GEIGY AG, 4002 Basel (CH)
(72) Erfinder: Meyer, Willy, CH-4125 Riehen (CH); Siegrist, Urs, CH-5268 Eiken (CH)

(56) Entgegenhaltungen:
- EP-A- 0 120 814
- EP-A- 0 248 245

## Beschreibung

Die vorliegende Erfindung betrifft ein Verfahren zur Herstellung von Benzolsulfonamiden der allgemeinen Formel I 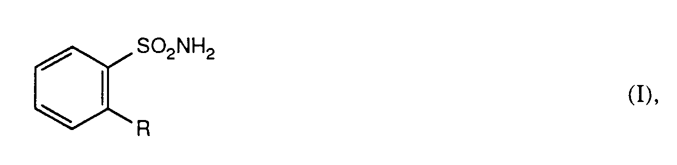
worin R 3,3-Difluorbutyl (-CH₂-CH₂-CF₂-CH₃) oder 3,3-Difluor-1-butenyl (-CH=CH-CF₂-CH₃) bedeutet.

Die Benzolsulfonamide der Formel I sind wertvolle Zwischenprodukte zur Herstellung von N-Phenylsulfonyl-N'-pyrimidinyl- bzw. -N'-triazinylsulfonylharnstoffen mit herbizider Wirkung. Solche Sulfonylharnstoffe sind beispielsweise in den Europäischen Patentanmeldungen EP-A-0 120 814 und EP-A-0 102 925 beschrieben. Die Benzolsulfonamide der Formel I eignen sich insbesondere zur Herstellung von N-[2-(3,3-Difluorbutyl)-phenyl]-N'-(4-methoxy-6-methyl-1,3,5-triazin-2-yl)-harnstoff und N-[2-(3,3-Difluor-1-butenyl)-phenyl]-N'-(4-methoxy-6-methyl-1,3,5-triazin-2-yl)-harnstoff.

Die Benzolsulfonamide der Formel I werden gemäss vorliegender Erfindung hergestellt, indem man ein 2-(3-Oxobuten-1-yl)-benzolsulfonsäuresalz der Formel II 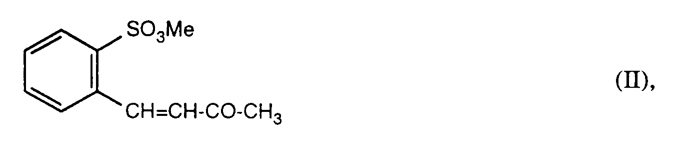
worin Me ein Alkalimetall- oder ein Ammoniumion bedeutet, durch Einwirkung von Wasserstoff in Gegenwart eines Katalysators in ein 2-(3-Oxobutyl)-benzolsulfonsäuresalz der Formel III 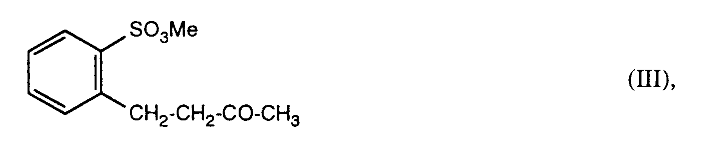
worin Me die obengenannte Bedeutung hat, überführt, dieses durch Umsetzung mit einem anorganischen Säurechlorid in 2-(3-Oxobutyl)-benzolsulfochlorid der Formel IV 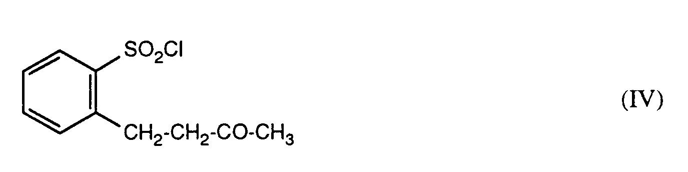
umwandelt, dieses dann durch Umsetzung mit Phosphorpentachlorid in ein Gemisch von Benzolsulfochloriden der Formel V 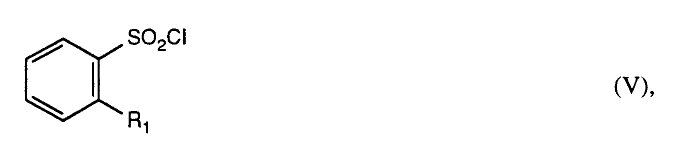
worin R₁ 3-Chlor-2-buten-1-yl 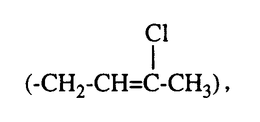
3-Chlor-3-buten-1-yl 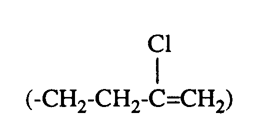
oder 3,3-Dichlorbutyl (-CH₂-CH₂-CCl₂-CH₃) bedeutet, überführt, dieses Gemisch mit Fluorwasserstoff zu einem Gemisch von 2-(3,3-Difluorbutyl)-benzolsulfohalogeniden der Formel VI 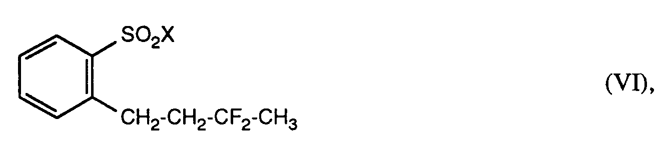
worin X Chlor oder Fluor bedeutet, umsetzt und dieses Gemisch entweder
a) durch Umsetzung, mit Ammoniak in 2-(3,3-Difluorbutyl)-benzolsulfonamid der Formel Ia 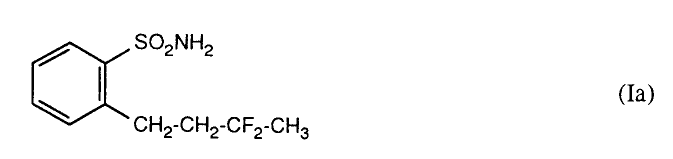 überführt, oder
b) zunächst mit einem Bromierungsmittel (1,3-Dibrom-5,5-dimethylhydantoin) zu einem Gemisch von 2-(1-Brom-3,3-difluorbutyl)-benzolsulfohalogeniden der Formel VII 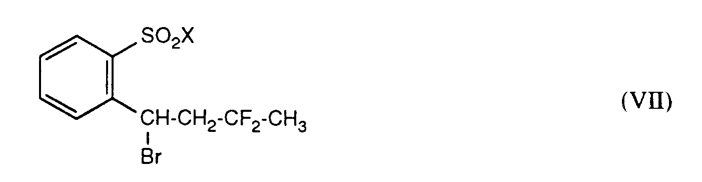 worin X Fluor oder Chlor bedeutet, umsetzt, dieses durch weitere Umsetzung mit Ammoniak in 2-(1-Brom-3,3-difluorbutyl)-sulfonamid der Formel VIII 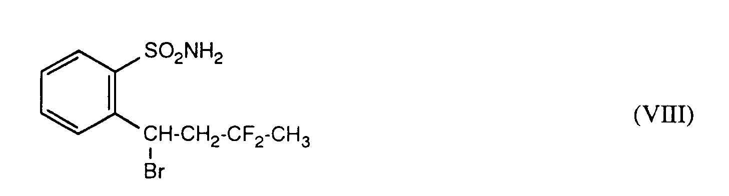 überführt und dieses dann durch Abspaltung von Bromwasserstoff in das 2-(3,3-Difluor-1-butenyl)-benzolsulfonamid der Formel Ib 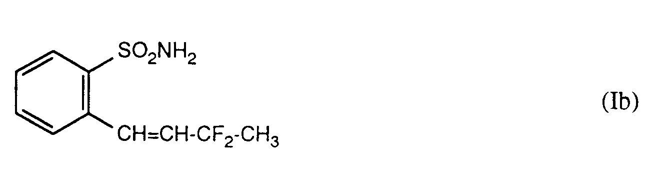 umwandelt.

Die als Ausgangsmaterialien dienenden Sulfonsäuresalze der Formel II können nach der in der Europäischen Patentanmeldung EP-A-0 102 925 beschriebenen Methode hergestellt werden, indem man Orthanilsäure zunächst diazotiert und das Diazoniumsalz anschliessend in Gegenwart eines Palladium-Katalysators mit Methylvinylketon umsetzt.

Die katalytische Hydrierung der Benzolsulfonsäuresalze der Formel II wird zweckmässig in einem inerten Lösungsmittel in Gegenwart eines Edelmetallkatalysators durchgeführt. Als Lösungsmittel kommen Wasser, Essigsäure und niedere Alkanole, insbesondere Methanol und Ethanol, in Betracht. Vorzugsweise wird die katalytische Hydrierung der Sulfonsäuresalze der Formel II in Wasser durchgeführt. Als Edelmetallkatalysatoren kommen in erster Linie Palladium auf Kohle oder Platin auf Kohle in Betracht. Die Reaktionstemperatur liegt in der Regel im Bereich von 0-50°C und der Druck im Bereich von 1-5 bar. Vorzugsweise wird die Reaktion bei Raumtemperatur und Normaldruck durchgeführt.

Bei der Ueberführung der Sulfonsäuresalze der Formel III in die entsprechenden Säurechloride kommen als anorganische Säurechloride Phosgen, Thionylchlorid und Phosphorpentachlorid in Betracht. Vorzugsweise werden die Sulfonsäuresalze der Formel III mit Phosgen umgesetzt. Diese Säurechloride werden in stöchiometrischer Menge oder im Ueberschuss eingesetzt. Das Molverhältnis von Sulfonsäuresalz der Formel III zu anorganischem Säurechlorid beträgt in der Regel 1:1-1,5. Die Reaktion wird zweckmässig in einem inerten organischen Lösungsmittel durchgeführt, wobei als Lösungsmittel insbesondere aliphatische und aromatische Kohlenwasserstoffe und Halogenkohlenwasserstoffe in Betracht kommen. Geeignete Lösungsmittel sind Hexan, Cyclohexan, Chloroform, Kohlenstofftetrachlorid, Toluol und Chlorbenzol. Vorzugsweise wird Chlorbenzol als Lösungsmittel verwendet. Die Umsetzung kann im Temperaturbereich von 20-130°C durchgeführt werden. Vorzugsweise wird die Umsetzung bei einer Temperatur von 70-90°C durchgeführt. Es ist vorteilhaft, die Umsetzung in Gegenwart einer katalytischen Menge N,N-Dimethylformamid durchzuführen.

Die Umsetzung des Sulfonsäurechlorids der Formel IV mit Phosphorpentachlorid wird vorteilhaft in einem inerten Lösungsmittel durchgeführt. Als Lösungsmittel kommen aliphatische und aromatische Kohlenwasserstoffe und Halogenkohlenwasserstoffe in Betracht. Geeignete Lösungsmittel sind Methylenchlorid, Chloroform, Kohlenstofftetrachlorid, Toluol und Chlorbenzol. Bevorzugt wird die Reaktion in Methylenchlorid als Lösungsmittel durchgeführt. Die Reaktionstemperatur kann im Bereich von 0-100°C variieren und liegt vorzugsweise bei 20-40°C. Das Phosphorpentachlorid wird in stöchiometrischer Menge oder in einem Ueberschuss von bis zu 20 % eingesetzt.

Die Umsetzung des Sulfochlorids der Formel IV mit Phosphorpentachlorid liefert ein Gemisch, das aus dem 2-(3-Chlor-2-buten-1-yl)-benzolsulfochlorid, dem 2-(3-Chlor-3-buten-1-yl)-benzolsulfochlorid und dem 2-(3,3-Dichlorbutyl)-benzolsulfochlorid besteht. Die Zusammensetzung des Gemisches ist von den gewählten Reaktionsbedingungen abhängig. In der Regel entsteht bei milden Reaktionsbedingungen mehr 2-(3,3-Dichlorbutyl)-benzolsulfochlorid, während bei schärferen Bedingungen die Bildung der 2-(3-Chlorbutenyl)-benzolsulfochloride bevorzugt ist. Dabei werden vom 2-(3-Chlor-2-buten-1-yl)-benzolsulfochlorid sowohl das cis- als auch das trans-Isomere gebildet. Das erhaltene Gemisch von Verbindungen der Formel V liefert jedoch bei der nachfolgenden Umsetzung mit Fluorwasserstoff in ausgezeichneter Ausbeute das 2-(3,3-Difluorbutyl)-benzolsulfohalogenid der Formel VI.

Die Umsetzung von Benzolsulfochloriden der Formel V mit Fluorwasserstoff wird vorteilhaft in einem inerten Lösungsmittel durchgeführt. Dabei dient entweder überschüssiger Fluorwasserstoff als alleiniges Lösungsmittel oder es kann zusätzlich ein weiteres inertes Lösungsmittel, wie n-Hexan, Cyclohexan, Toluol oder Chlorbenzol, verwendet werden. Die Reaktionstemperatur kann in weiten Grenzen variieren. Geeignete Reaktionstemperaturen liegen im Bereich von -50-+100°C. Vorzugsweise wird die Reaktion bei einer Temperatur von 0-50°C durchgeführt. Zur Durchführung der Reaktion geht man zweckmässig so vor, dass man die Reaktanten bei tiefer Temperatur mischt und dann in einem geschlossenen System auf Reaktionstemperatur aufheizt. Dabei baut sich in Folge der Bildung von Chlorwasserstoff ein erhöhter Druck im Reaktionsgefäss auf. Da dieser Chlorwasserstoff zu Nebenreaktionen Anlass geben kann, ist es vorteilhaft, den Chlorwasserstoff von Zeit zu Zeit aus dem Reaktionsgemisch zu entfernen.

Die Umsetzung des Benzolsulfohalogenids der Formel VI mit Ammoniak wird zweckmässig in der Weise durchgeführt, dass man Ammoniak in eine Lösung des Sulfohalogenids der Formel VI in einem inerten Lösungsmittel einleitet. Als Lösungsmittel kommen aliphatische und aromatische Kohlenwasserstoffe, Halogenkohlenwasserstoffe und Ether in Betracht. Als geeignete Lösungsmittel sind beispielsweise n-Hexan, Cyclohexan, Toluol, Chlorbenzol, Diethylether, Tetrahydrofuran oder Dioxan genannt. Die Reaktionstemperatur kann zwischen Raumtemperatur und 50°C liegen. Die Reaktion kann auch in einem zweiphasigen Reaktionsmedium in der Weise durchgeführt werden, dass man beispielsweise eine Lösung des Sulfohalogenids der Formel VI in Toluol mit wässriger Ammoniaklösung umsetzt.

Die Bromierung des Sulfohalogenids der Formel VI wird vorzugsweise in einem inerten Lösungsmittel, z.B. einem aliphatischen oder aromatischen Kohlenwasserstoff oder Halogenkohlenwasserstoff durchgeführt. Bevorzugte Lösungsmittel sind aliphatische halogenierte Kohlenwasserstoffe, wie Chloroform und Kohlenstofftetrachlorid. Als Bromierungsmittel sind Brom, und Brom abspaltende Substanzen wie N-Bromsuccinimid und 1,3-Dibrom-5,5-dimethylhydantoin in Betracht. Vorzugsweise wird die Bromierung mit 1,3-Dibrom-5,5-dimethylhydantoin durchgeführt. Das Bromierungsmittel wird in äquivalenter Menge oder einem Ueberschuss von bis zu 20 % eingesetzt. Es ist vorteilhaft, die Bromierungsreaktion in Gegenwart eines Radikalbildners, wie UV-Licht oder Azoisobutyronitril durchzuführen.

Die weitere Umsetzung des Benzolsulfohalogenids der Formel VII mit Ammoniak erfolgt vorteilhaft in einem inerten Lösungsmittel, wie einem Ether oder einem Kohlenwasserstoff. Geeignete Lösungsmittel sind Diethylether, Tetrahydrofuran, Dioxan, Pentan, Hexan, Cyclohexan und ferner Acetonitril. Die Reaktion wird vorteilhaft in der Weise durchgeführt, dass man Ammoniak in eine Lösung des Benzolsulfohalogenids der Formel VII in einem der vorgenannten Lösungsmittel einleitet. Dabei wird das Ammoniak in stöchiometrischer Menge oder in einem geringfügigen Ueberschuss von bis zu 10 % eingesetzt.

Die bei der Durchführung des erfindungsgemässen Verfahrens durchlaufenen Zwischenprodukte der Formeln III, IV, V, VI, VII und VIII sind neue Verbindungen und ebenfalls Gegenstand der vorliegenden Erfindung.

Durch das erfindungsgemässe Verfahren wird es möglich, die Benzolsulfonamide der Formel I ausgehend von leicht zugänglichen Ausgangsmaterialien in einfacher Weise und in guter Ausbeute herzustellen. Die Benzolsulfonamide der Formel I können in an sich bekannter Weise, beispielsweise durch Umsetzung mit 2-Alkoxycarbonylamino- beziehungsweise 2-Phenoxycarbonylaminopyrrimidenen bzw. -triazinen, in Sulfonylharnstoffe mit herbizider Wirkung übergeführt werden.

Das erfindungsgemässe Verfahren wird durch die nachfolgenden Beispiele näher erläutert.

### Beispiel 1: Herstellung von 2-(3-Oxobutyl)-benzolsulfonsäure-Natriumsalz

325 g 2-(3-Oxobuten-1-yl)-benzolsulfonsäure-Natriumsalz (73%ig) werden in 2700 ml Wasser gelöst und nach Zugabe von 20 g eines Platin/Kohle-Katalysators (5%ig) mit Wasserstoff geschüttelt. Die Wasserstoffaufnahme kommt nach 2 Stunden zum Stillstand. Es wurden 20,5 l Wasserstoff aufgenommen. Danach wird der Katalysator abfiltriert und das Filtrat zur Trockene eingedampft. Der Rückstand wird mit Chlorbenzol aufgenommen und durch azeotrope Destillation vom Restwasser befreit. Die so erhaltene Suspension von 2-(3-Oxobutyl)-benzolsulfonsäure-Natriumsalz kann direkt für die nachfolgende Umsetzung mit Phosgen verwendet werden.

### Beispiel 2: Herstellung von 2-(3-Oxobutyl)-benzolsulfochlorid

In die nach Beispiel 1 erhaltene Suspension von 2-(3-Oxobutyl)-benzolsulfonsäure-Natriumsalz in Chlorbenzol wird nach Zugabe von 10 ml Dimethylformamid bei 75-80°C während 5 Stunden 197 g Phosgen eingeleitet. Dann wird während 2 Stunden Stickstoff durch das Reaktionsgemisch geleitet, wobei die Temperatur auf 20-25°C sinkt. Das Reaktionsgemisch wird dann filtriert, zur Trockene eingedampft und der Rückstand aus Cyclohexan umkristallisiert. Man erhält 163 g 2-(3-Oxobutyl)-benzolsulfochlorid vom Schmelzpunkt 67-68°C.

### Beispiel 3: Herstellung eines aus 2-(3,3-Dichlorbutyl)-benzolsulfochlorid, 2-(3-Chlor-2-buten-1-yl)-benzolsulfochlorid und 2-(3-Chlor-3-buten-1-yl)-benzolsulfochlorid bestehenden Gemisches.

Eine Lösung von 116 g 2-(3-Oxobutyl)-benzolsulfochlorid in 700 ml Dichlormethan wird bei 20 - 30°C während 1 Stunde portionsweise mit 102,7 g Phosphorpentachlorid versetzt und anschliessend 15 Stunden bei 20 - 25°C gerührt. Dann wird das Reaktionsgemisch zur Trockene eingedampft und der Rückstand mit Chlorbenzol aufgenommen. Durch erneutes Eindampfen zur Trockene erhält man 118 g eines Gemisches, das nach gaschromatographischer Analyse zu etwa 20 % aus 2-(3,3-Dichlorbutyl)-benzolsulfochlorid, 35 % 2-(3-Chlor-3-buten-1-yl)-benzolsulfochlorid und 45 % der cis/trans-isomeren Formen von 2-(3-Chlor-2-buten-1-yl)-benzolsulfochlorid besteht.

### Beispiel 4: Herstellung von 2-(3,3-Difluorbutyl)-benzolsulfochlorid

42,3 g des nach Beispiel 3 hergestellten Gemisches von Benzolsulfochloriden werden in einem Autoklaven bei -50°C mit 90 g Fluorwasserstoff versetzt. Dann wird auf 18°C erwärmt und während 24 Stunden bei 18 - 19°C gerührt, wobei von Zeit zu Zeit der gebildete Chlorwasserstoff aus dem Autoklaven abgelassen wird. Nach beendigter Umsetzung wird der überschüssige Fluorwasserstoff abgesaugt, der Rückstand in Dichlormethan gelöst, die Lösung zweimal mit Eiswasser gewaschen und über Natriumsulfat getrocknet. Durch Eindampfen, Filtrieren des Rückstandes durch eine Kieselgelschicht mit einem Gemisch von 1 Teil Toluol und 4 Teilen Hexan und Eindampfen des Filtrats zur Trockene erhält man 20,4 g 2-(3,3-Difluorbutyl)-benzolsulfochlorid als Öl. Das Produkt enthält gemäss ¹H-NMR-spektroskopischer Analyse etwa 5 Gew.-% 2-(3,3-Difluorbutyl)-benzolsulfofluorid.

### Beispiel 5: Herstellung von 2-(3,3-Difluorbutyl)-benzolsulfonamid

Eine Mischung von 50 ml 30%ige Ammoniaklösung und 50 ml Wasser werden bei 15-20°C tropfenweise mit einer Lösung von 26,9 g 2-(3,3-Difluorbutyl)-benzolsulfochlorid und 200 ml Methylenchlorid versetzt und anschliessend während 3 Stunden bei 20-25°C nachgerührt. Durch Abtrennen der Methylenchloridphase, Waschen derselbigen mit Wasser, Trocknen über Natriumsulfat, Eindampfen und Umkristallisieren des Rückstandes aus Toluol erhält man 19,9 g 2-(3,3-Difluorbutyl)-benzolsulfonamid vom Schmelzpunkt 70-72°C.

### Beispiel 6: Herstellung von 2-(1-Brom-3,3-difluorbutyl)-benzolsulfochlorid

Eine Mischung von 13,4 g 2-(3,3-Difluorbutyl)-benzolsulfochlorid, 8,6 g 1,3-Dibrom-5,5-dimethylhydantoin und 80 ml Kohlenstofftetrachlorid wird 3 Stunden bei 75 - 80°C gerührt und dabei mit einer Quecksilberdampflampe bestrahlt. Dann wird das Reaktionsgemisch zur Trockene eingedampft, der Rückstand mit Hexan aufgenommen, filtriert und das Filtrat erneut zur Trockene eingedampft. Man erhält 15,3 g 2-(1-Brom-3,3-difluorbutyl)-benzolsulfochlorid als hellgelbes Öl, welches aufgrund der ¹H-NMR-spektroskopischer Analyse etwa 5 Gew.-% 2-(1-Brom-3,3-difluorbutyl)-benzolsulfofluorid enthält.

### Beispiel 7: Herstellung von 2-(1-Brom-3,3-difluorbutyl)-benzolsulfonamid

In eine Lösung, von 13,9 g des nach Beispiel 6 hergestellten 2-(1-Brom-3,3-difluorbutyl)-benzolsulfochlorids in 50 ml Diethylether werden bei -5- 0°C 1,5 g Ammoniak eingeleitet. Dann wird das Reaktionsgemisch zunächst mit Eiswasser und dann mit 10%iger Salzsäure versetzt. Die organische Phase wird abgetrennt, mit Wasser gewaschen und getrocknet. Nach Abdampfen des Lösungsmittels erhält man 11,8 g 2-(1-Brom-3,3-difluorbutyl)-benzolsulfonamid vom Schmelzpunkt 104-105°C.

### Beispiel 8: Herstellung von 2-(3,3-Difluorbuten-1-yl)-benzolsulfonamid

Eine Lösung von 3,3 g 2-(1-Brom-3,3-difluorbutyl)-benzolsulfonamid und 30 ml Dioxan wird bei 20-25°C tropfenweise mit einer Lösung von 1,52 g 1,8-Diazabicyclo[5.4.0]-undec-7-en(1,5-5) und 10 ml Dioxan versetzt und anschliessend während 2 Stunden nachgerührt. Durch Eingiessen in Wasser, Ansäuern mit 10%iger Salzsäure, Extrahieren mit Essigester, Waschen mit Wasser, Trocknen über Natriumsulfat, Eindampfen und Reinigen des Rückstandes über eine Kieselgel-Säule erhält man 0,7 g 2-(3,3-Difluorbuten-1-yl)-benzolsulfonamid vom Schmelzpunkt 103-104°C.

## Patentansprüche

1. Verfahren zur Herstellung von Benzolsulfonamiden der allgemeinen Formel I 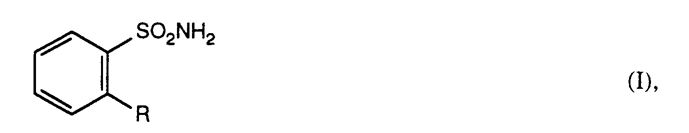 worin R 3,3-Difluorbutyl (-CH₂-CH₂-CF₂-CH₃) oder 3,3-Difluor-1-butenyl (-CH=CH-CF₂-CH₃) bedeutet, dadurch gekennzeichnet, dass man ein 2-(3-Oxobuten-1-yl)-benzolsulfonsäuresalz der Formel II 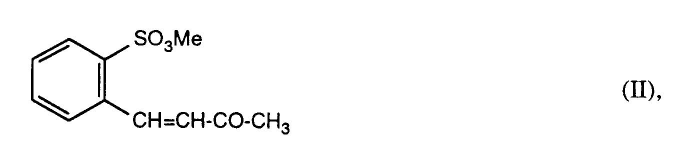 worin Me ein Alkalimetall- oder ein Ammoniumion bedeutet, durch Einwirkung von Wasserstoff in Gegenwart eines Katalysators in ein 2-(3-Oxobutyl)-benzolsulfonsäuresalz der Formel III 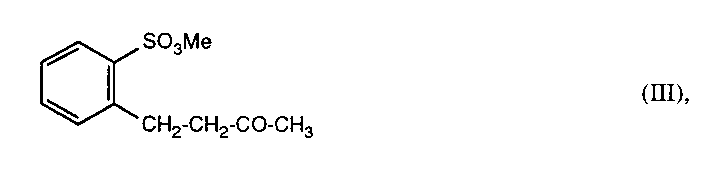 worin Me die obengenannte Bedeutung hat, überführt, dieses durch Umsetzung mit einem anorganischen Säurechlorid in 2-(3-Oxobutyl)-benzolsulfochlorid der Formel IV 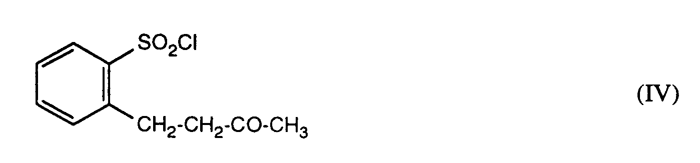 umwandelt, dieses dann durch Umsetzung mit Phosphorpentachlorid in ein Gemisch von Benzolsulfochloriden der Formel V 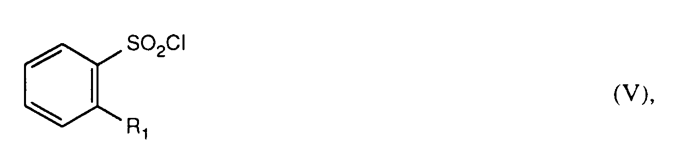 worin R₁ 3-Chlor-2-buten-1-yl 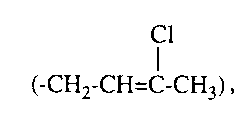 3-Chlor-3-buten-1-yl 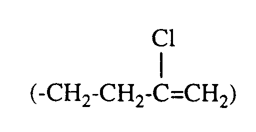 oder 3,3-Dichlorbutyl (-CH₂-CH₂-CCl₂-CH₃) bedeutet, überführt, dieses Gemisch mit Fluorwasserstoff zu einem Gemisch von 2-(3,3-Difluorbutyl)-benzolsulfohalogeniden der Formel VI 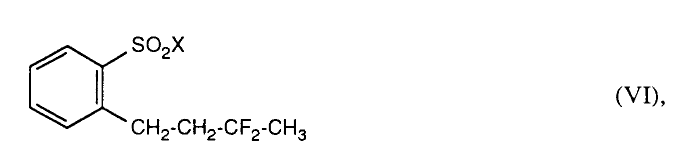 worin X Chlor oder Fluor bedeutet, umsetzt und dieses Gemisch entweder
a) durch Umsetzung mit Ammoniak in 2-(3,3-Difluorbutyl)-benzolsulfonamid der Formel Ia 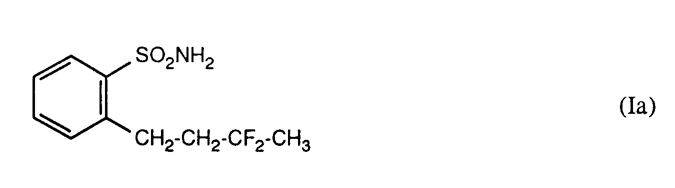 überführt, oder
b) zunächst mit einem Bromierungsmittel zu einem Gemisch von 2-(1-Brom-3,3-difluorbutyl)-benzolsulfohalogeniden der Formel VII 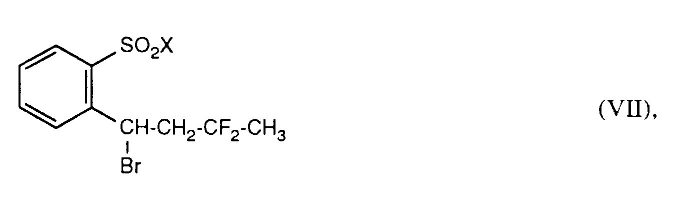 worin X Fluor oder Chlor bedeutet, umsetzt, dieses durch weitere Umsetzung mit Ammoniak in 2-(1-Brom-3,3-difluorbutyl)-sulfonamid der Formel VIII 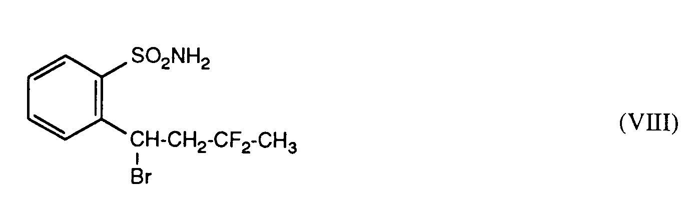 überführt und dieses dann durch Abspaltung von Bromwasserstoff in das 2-(3,3-Difluor-1-butenyl)-benzolsulfonamid der Formel Ib 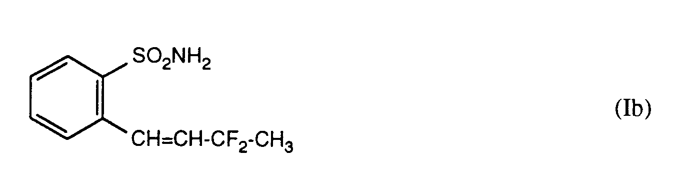 umwandelt.

2. Verfahren nach Anspruch 1, dadurch gekennzeichent, dass man die katalytische Hydrierung der Benzolsulfonsäuresalze der Formel II in Wasser als Lösungsmittel bei einer Temperatur von 0-50°C und einem Druck von 1 - 5 bar in Gegenwart eines Palladium- oder Platinkatalysators durchführt.

3. Verfahren nach Anspruch 1, dadurch gekennzeichnet, dass man die Sulfonsäuresalze der Formel III in Chlorbenzol als Lösungsmittel bei einer Temperatur von 70-90°C in Gegenwart einer katalytischen Menge N,N-Dimethylformamid mit Phosgen umsetzt.

4. Verfahren nach Anspruch 1, dadurch gekennzeichnet, dass man die Umsetzung des Sulfonsäurechlorids der Formel IV mit Phosphorpentachlorid in Methylenchlorid als Lösungsmittel bei einer Temperatur von 20-40°C durchführt.

5. Verfahren nach Anspruch 1, dadurch gekennzeichnet, dass man die Umsetzung der Benzolsulfochloride der Formel V mit Fluorwasserstoff bei einer Temperatur von 0-50°C durchführt.

6. Verfahren nach Anspruch 1, dadurch gekennzeichnet, dass man die Umsetzung der Benzolsulfohalogenide der Formeln VI oder VII in der Weise durchführt, dass man in eine Lösung dieser Sulfohalogenide in einem inerten Lösungsmittel bei einer Temperatur zwischen Raumtemperatur und 50°C Ammoniak einleitet.

7. Verfahren nach Anspruch 1, dadurch gekennzeichnet, dass man die Bromierung des Sulfohalogenids der Formel VI in einem aliphatischen halogenierten Kohlenwasserstoff als Lösungsmittel mit 1,3-Dibrom-5,5-dimethylhydantoin in Gegenwart eines Radikalbildners durchführt.

8. Sulfonsäuresalze der Formel III 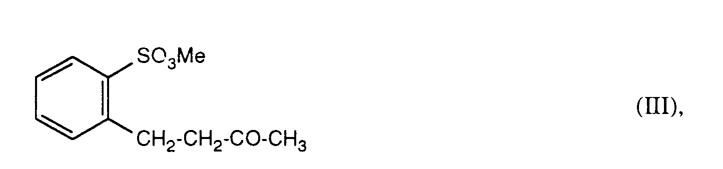 in welcher Me ein Alkalimetall - oder Ammoniumion bedeutet.

9. 2-(3-Oxobutyl)-benzolsulfochlorid der Formel IV

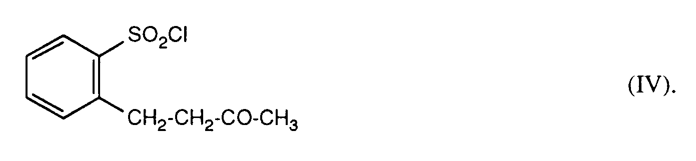

10. Benzolsulfochloride der Formel V 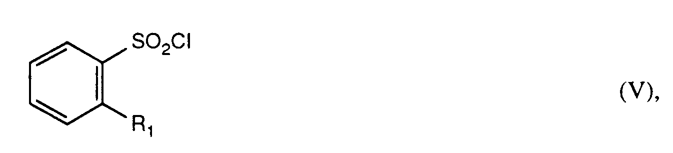 worin R₁ 3-Chlor-2-buten-1-yl 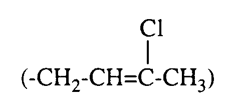 bedeutet.

11. 2-(3,3-Difluorbutyl)-benzolsulfohalogenide der Formel VI 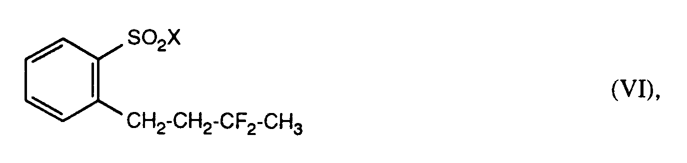 worin X Fluor bedeutet.

12. 2-(1-Brom-3,3-difluorbutyl)-benzolsulfohalogenide der Formel VII 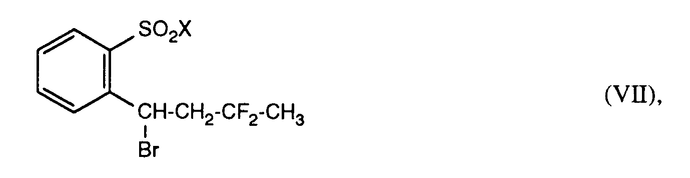 worin X Fluor oder Chlor bedeutet.

13. 2-(1-Brom-3,3-difluorbutyl)-benzolsulfonamid der Formel VIII

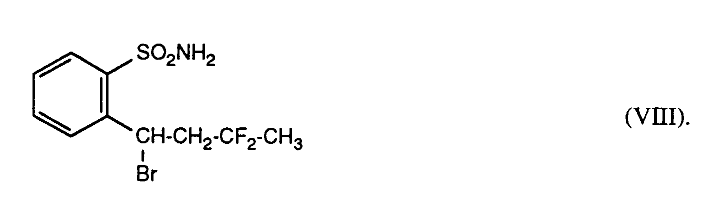

## Claims

1. A process for the preparation of a benzenesulfonamide of general formula I 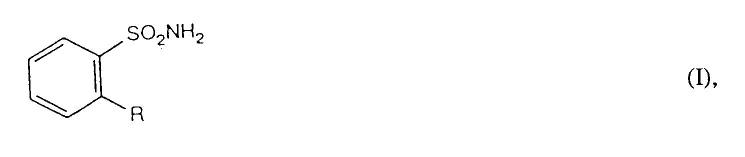 wherein R is 3,3-difluorobutyl (-CH₂-CH₂-CF₂-CH₃) or 3,3-difluorobuten-1-yl (-CH=CH-CF₂-CH₃), which comprises converting a 2-(3-oxobuten-1-yl)benzenesulfonic acid salt of formula II 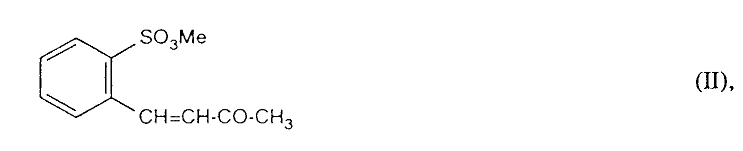 wherein Me is an alkali metal ion or an ammonium ion, by catalytic hydrogenation into a 2-(3-oxobutyl)benzenesulfonic acid salt of formula III 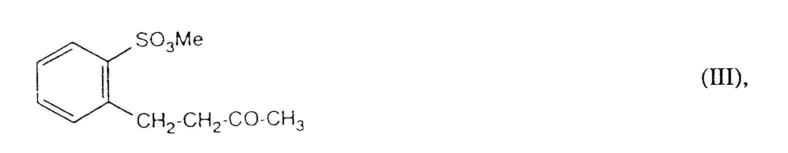 wherein Me has the above meaning, converting said acid salt of formula III by reaction with an inorganic acid chloride into a 2-(3-oxobutyl)benzenesulfonyl chloride of formula IV 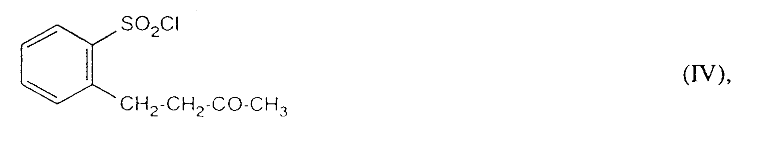 then converting said sulfonyl chloride by reaction with phosphorus pentachloride into a mixture of benzenesulfonyl chlorides of formula V 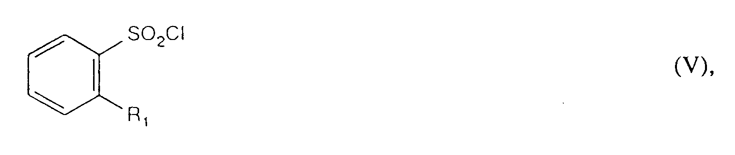 wherein R₁ is 3-chloro-2-buten-1-yl 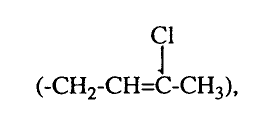 3-chloro-3-buten-1-yl 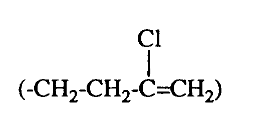 or 3,3-dichlorobutyl (-CH₂-CH₂-CCl₂-CH₃), reacting said mixture with hydrogen fluoride to give a mixture of 2-(3,3-difluorobutyl)-benzenesulfonyl halides of formula VI 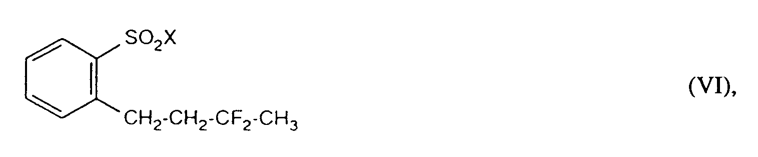 wherein X is chloro or fluoro, and converting said mixture either
a) by reaction with ammonia into the 2-(3,3-difluorobutyl)benzenesulfonamide of formula Ia 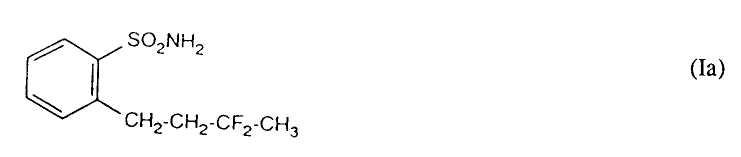 or
b) by reaction initially with a brominating agent into a mixture of 2-(1-bromo-3,3-difluorobutyl)benzenesulfonyl halides of formula VII 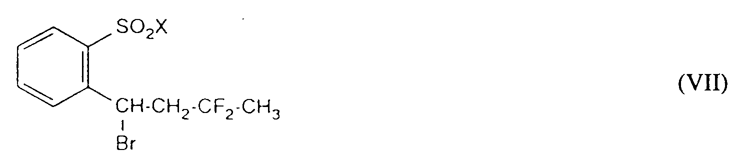 wherein X is fluoro or chloro, and then converting this mixture by further reaction with ammonia into the 2-(1-bromo-3,3-difluorobutyl)sulfonamide of formula VIII 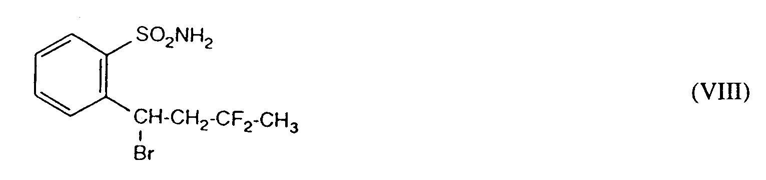 which is then dehydrobrominated to the 2-(3,3-difluorobuten-1-yl)benzenesulfonamide of formula Ib

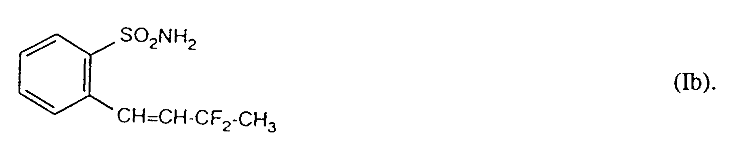

2. A process according to claim 1, wherein the catalytic hydrogenation of the benzenesulfonic acid salt of formula II is carried out in water as solvent in the temperature range from 0-50°C and under a pressure of 1-5 bar in the presence of a palladium or platinum catalyst.

3. A process according to claim 1, wherein the sulfonic acid salt of formula III is reacted with phosgene in chlorobenzene as solvent in the temperature range from 70-90°C in the presence of a catalytic amount of N,N-dimethylformamide.

4. A process according to claim 1, wherein the reaction of the sulfonyl chloride of formula IV with phosphorus pentachloride is carried out in methylene chloride as solvent in the temperature range from 20-40°C.

5. A process according to claim 1, wherein the reaction of the benzenesulfonyl chlorides of formula V with hydrogen fluoride is carried out in the temperature range from 0-50°C.

6. A process according to claim 1, wherein the reaction of the benzenesulfonyl halides of formula VI or VII is carried out by introducing ammonia into a solution of said sulfonyl halides in an inert solvent in the temperature range from room temperature to 50°C.

7. A process according to claim 1, wherein the bromination of the sulfonyl halide of formula VI is carried out in an aliphatic halogenated hydrocarbon as solvent with 1,3-dibromo-5,5-dimethylhydantoin in the presence of a radical former.

8. A sulfonic acid salt of formula III 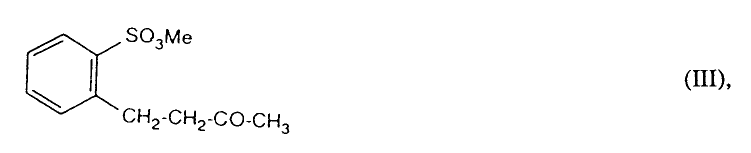 wherein Me is an alkali metal ion or an ammonium ion.

9. A 2-(3-oxobutyl)benzenesulfonyl chloride of formula IV

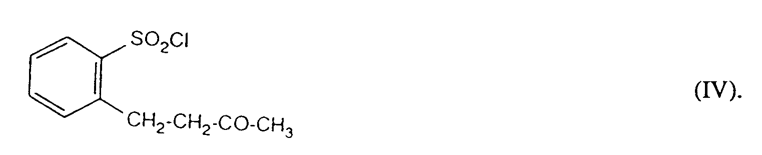

10. A benzenesulfonyl chloride of formula V 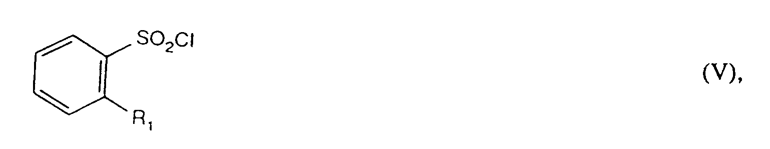 wherein R₁ is 3-chloro-2-buten-1-yl

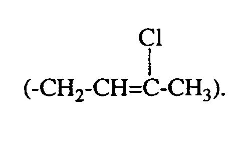

11. A 2-(3,3-difluorobutyl)benzenesulfonyl halide of formula VI 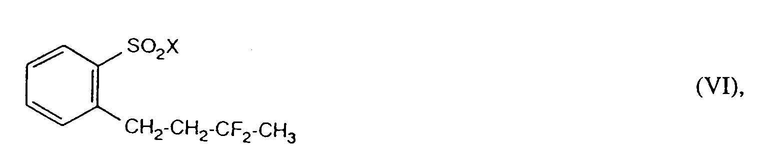 wherein X is fluoro.

12. A 2-(1-bromo-3,3-difluorobutyl)benzenesulfonyl halide of formula VII 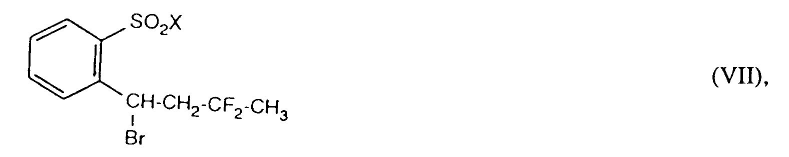 wherein X is fluoro or chloro.

13. A 2-(1-bromo-3,3-difluorobutyl)benzenesulfonamide of formula VIII

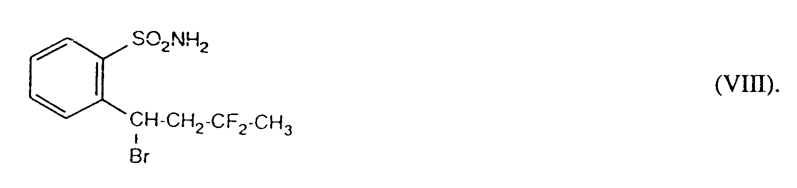

## Revendications

1. Procédé pour la préparation de benzènesulfonlamides de formule générale I 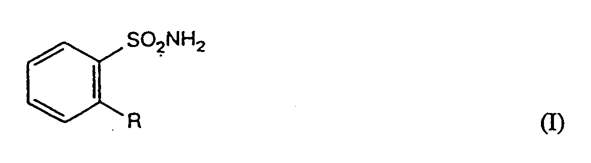 où R représente le 3,3-difluorobutyle (-CH₂-CH₂-CF₂-CH₃) ou le 3,3-difluoro-1-butényle (-CH=CH-CF₂-CH₃), caractérisé en ce que l'on transforme un sel de l'acide 2-(3-oxobutén-1-yl)-benzènesulfonique de formule II 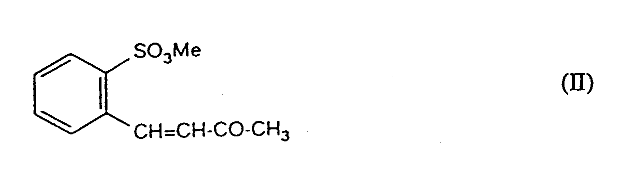 où Me signifie un ion métal alcalin ou un ion ammonium, par action de l'hydrogène en présence d'un catalyseur en un sel de l'acide 2-(3-oxobutyl)-benzènesulfonique de formule III 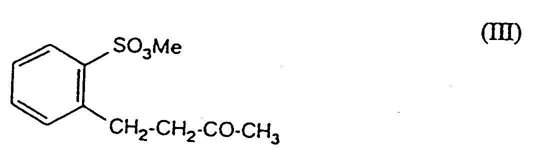 où Me a la signification donnée ci-dessus, en ce que l'on transforme celui-ci par réaction avec un chlorure d'acide minéral en 2-(3-oxobutyl)-benzène-sulfochlorure de formule IV 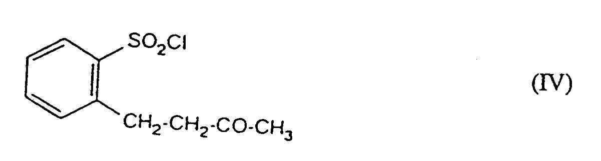 puis en ce que l'on transforme celui-ci par réaction avec du pentachlorure de phosphore en un mélange de benzène-sulfochlorures de formule 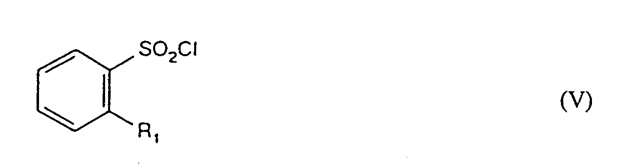 où R₁ représente le 3-chloro-2-butén-1-yle 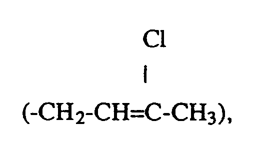 le 3-chloro-3-butén-1-yle 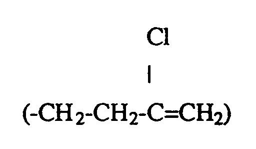 ou le 3,3-dichlorobutyl (-CH₂-CH₂-CCl₂-CH₃), en ce que l'on fait réagir ce mélange avec l'acide fluorhydrique pour former un mélange de 2-(3,3-difluorobutyl)-benzène-sulfohalogénures de formule VI 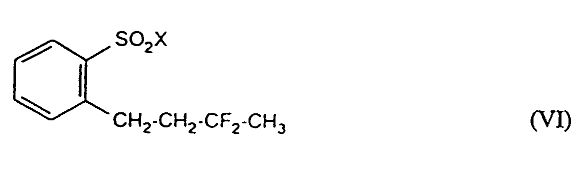 où X représente le chlore ou le fluor et ou
a) en transformant ce mélange par réaction avec l'ammoniac en 2-(3,3-difluorobutyl)-benzènesulfonamide de formule Ia 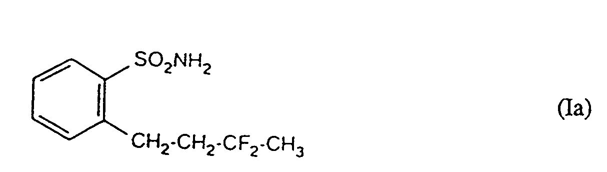
b) en faisant tout d'abord réagir ce mélange avec un agent de bromation pour former un mélange de 2-(1-bromo-3,3-difluorobutyl)-benzène-sulfohalogénures de formule VII 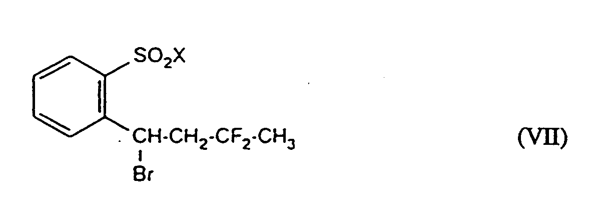 où X renrésente le fluor ou le chlore, en ce que l'on transforme celui-ci par réaction avec l'ammoniac en 2-(1-bromo-3,3-difluorobutyl)-sulfonamide de formule VIII 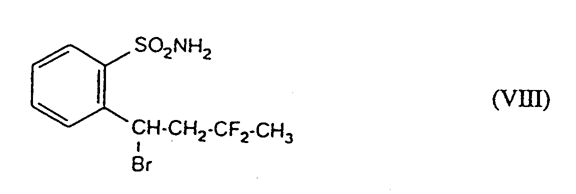 puis en ce que l'on transforme celui-ci par clivage de l'acide bromhydrique en 2-(3,3-difluoro-1-butényl)-benzènesulfonamide de formule Ib

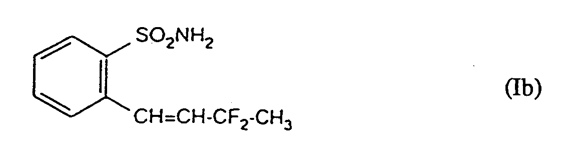

2. Procédé selon la revendication 1, caractérisé en ce que l'on effectue l'hydrogénation catalytique des sels de l'acide benzènesulfonique de formule II dans l'eau comme solvant à une température comprise entre 0 et 50°C et sous une pression comprise entre 1 et 5 bars en présence d'un catalyseur au palladium ou au platine.

3. Procédé selon la revendication 1, caractérisé en ce que l'on fait réagir avec du phosgène les sels de l'acide sulfonique de formule III dans du chlorobenzène comme solvant à une température comprise entre 70 et 90°C en présence d'une quantité catalytique de N,N-diméthylformamide.

4. Procédé selon la revendication 1, caractérisé en ce que l'on effectue la réaction du chlorure de l'acide sulfonique de formule IV avec du pentachlorure de phosphore dans du chlorure de méthylène comme solvant à une température comprise entre 20 et 40°C.

5. Procédé selon la revendication 1, caractérisé en ce que l'on effectue la réaction des benzène-sulfochlorures de formule V avec l'acide fluorhydrique à une température comprise entre 0 et 50°C.

6. Procédé selon la revendication 1, caractérisé en ce que l'on effectue la transformation des benzène-sulfohalogénures de formule VI ou VII, en introduisant de l'ammoniac dans une solution de ces sulfohalogénures dans un solvant inerte à une température comprise entre la température ambiante et 50°C.

7. Procédé selon la revendication 1, caractérisé en ce que l'on effectue la bromation du sulfohalogénure de formule VI dans un hydrocarbure aliphatique halogéné comme solvant avec la 1,3-dibromo-5,5-diméthylhydantoïne en présence d'un générateur de radicaux.

8. Sels de l'acide sulfonique de formule III 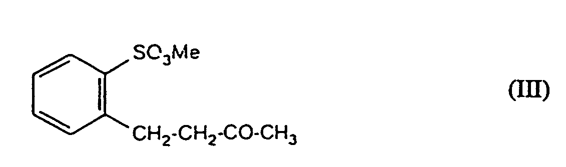 dans laquelle Me représente un ion métal alcalin ou un ion ammonium.

9. Le 2-(3-oxobutyl)-benzène-sulfochlorure de formule IV

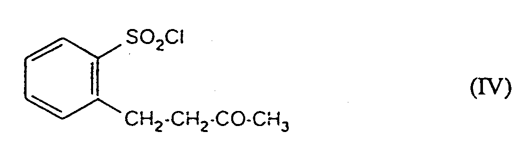

10. Les benzène-sulfochlorures de formule V 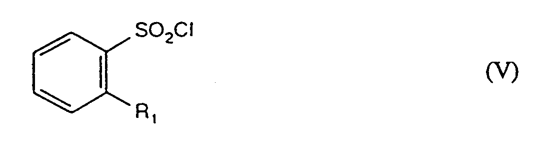 où R₁ représente le 3-chloro-2-butén-1-yle

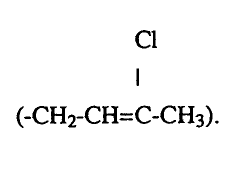

11. Les 2-(3,3-difluorobutyl)-benzène-sulfohalogénures de formule VI 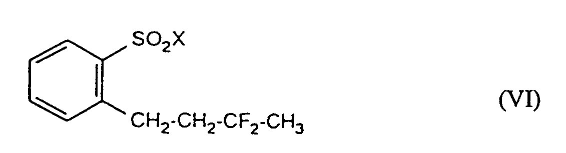 où X représente le fluor.

12. Les 2-(1-bromo-3,3-difluorobutyl)-benzène-sulfohalogénures de formule VII 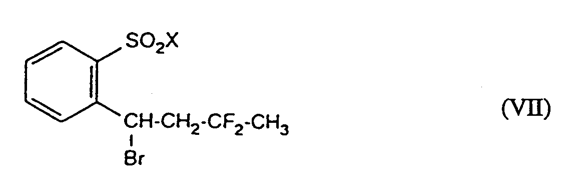 où X représente le fluor ou le chlore.

13. Le 2-(1-bromo-3,3-difluorobutyl)-benzènesulfonamide de formule VIII
